(19) Europäisches Patentamt
European Patent Office
Office européen des brevets

(11) EP 1 304 102 A2

(12) **EUROPÄISCHE PATENTANMELDUNG**

(43) Veröffentlichungstag:
**23.04.2003 Patentblatt 2003/17**

(51) Int Cl.[7]: **A61K 7/48**, A61K 7/42

(21) Anmeldenummer: **02023277.3**

(22) Anmeldetag: **17.10.2002**

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **17.10.2001 DE 10151245**

(71) Anmelder: **Beiersdorf AG**
**20245 Hamburg (DE)**

(72) Erfinder:
- **Raschke, Thomas, Dr.**
  **22529 Pinneberg (DE)**
- **Schwanke, Frank, Dr.**
  **20253 Hamburg (DE)**

(54) **Kombination von Retinoiden und Cyclodextrinen in kosmetischen oder dermatologischen O/W-Emulsionen**

(57) Kosmetische und/oder dermatologische O/W-Emulsion mit einem Gehalt an Retinoiden, Glycerylstearat und Cyclodextrinen.

EP 1 304 102 A2

Printed by Jouve, 75001 PARIS (FR)

## Beschreibung

Die vorliegende Erfindung betrifft Wirkstoffkombinationen zur Herstellung von kosmetischen oder dermatologischen Zubereitungen zur Prophylaxe und Behandlung von entzündlichen Hautzuständen und/oder zum Hautschutz bei empfindlich determinierter trockener Haut.

Ferner betrifft die Erfindung die Verwendung solcher Wirkstoffe und Zubereitungen, solche Wirkstoffe enthaltend, zur Immunstimulation der Haut, dabei vorteilhaft auch zur Immunstimulation im Sinne einer Behandlung der verletzten Haut, insbesondere zur Behandlung von Wunden.

Darüber hinaus betrifft.die Erfindung Zubereitungen mit extrem niedrigem sogenanntem "Stinging Potential" sowie kosmetische oder dermatologische Formulierungen, welche die Haut - z. B. nach einem Sonnenbad - gezielt pflegen und die Nachreaktionen der Haut auf die Einwirkung von UV-Strahlung vermindern.

Grundsätzlich besteht bei der Anwendung lipophiler und schwerlöslicher Wirkstoffe auf der Haut das Problem, daß diese oft nur zum Teil in die Haut eindringen und so ihre volle Wirkung nicht genutzt werden kann. Es hat nicht an Versuchen gefehlt, diesem Umstand abzuhelfen. Es wurde beispielsweise versucht, derartige Wirkstoffe in Liposomen zu verkapseln, durch Ethanolzugabe die Löslichkeit von Retinoiden zu verbessern oder Wirkstoffe in Mikroemulsionen einzuarbeiten.

Retinoide sind Isoprenoide mit 20 Kohlenstoffatomen mit einem typischen unpolaren Cyklus an einem Molekülende. Die namensgebede Substanz Retinol kommt in sehr hohen Anteilen im Fischöl vor, wird aber heute synthetisch hergestellt und in großen Mengen z.B. als Futtermittelzusatz verwendet.

Als Retinoide im Sinne der vorliegenden Schrift werden begrifflich auch alle kosmetisch und/oder pharmazeutisch unbedenklichen Retinoide, einschließlich des Retinols und seiner Ester, des Retinals sowie der Retinoäsäure und deren Ester einbezogen.

Retinol ist durch folgende Struktur gekennzeichnet:

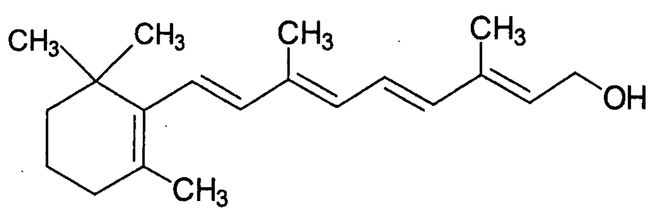

Retinol (auch: Axerophthol; [3,7-Dimethyl-9-(2,6,6-trimethyl-1-cyclohexenyl)-2,4,6,8-nonatetraen-1-ol) ist synonym mit Vitamin $A_1$, wird auch analog den Derivaten Retin-1-carbonsäure (Vitamin-A-Säure, Retinoësäure, Tretinoin) und deren Estern bzw. dem Retin-1-al (Vitamin-A-aldehyd) gelegentlich Vitamin-A-Alkohol genannt.

Bedeutend sind auch Retinolester, die durch folgende Struktur gekennzeichnet sind:

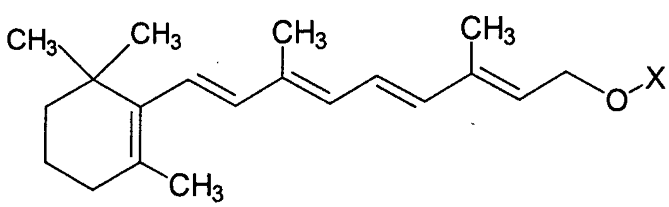

,

wobei X bevorzugt einen verzweigten oder unverzweigten Alkanoyl- oder Alkenoylrest darstellt. Retinal ist durch die Struktur

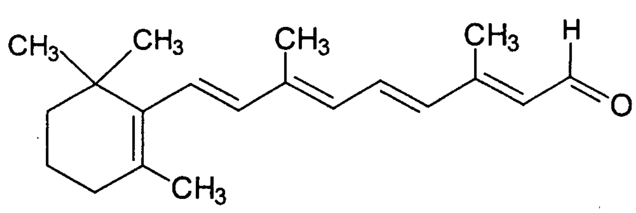

gekennzeichnet. Retinal [Vitamin A1-Aldehyd, 3,7-Dimethyl-9-(2,6,6-trimethyl-1-cyclohexenyl)- 2,4,6,8- nonatetraenal] ist in seiner all-trans-Form am stabilsten. Das früher Retinen genannte Retinal bildet, an Opsine gebunden, die Sehpigmente Rhodopsin und Iodopsin sowie das andere Funktionen wahrnehmende Bakteriorhodopsin. Retinal entsteht durch oxidative Spaltung des Carotins.

Retinoesäure [Vitamin-A-Säure, all-trans- 3,7-Dimethyl-9-(2,6,6-trimethyl- 1- cyclohexenyl)-2,4,6,8- nonatetraensäure] ist durch die Struktur

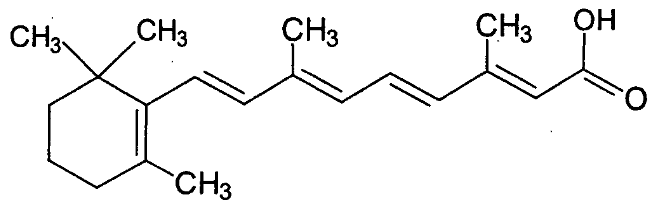

gekennzeichnet. Sie ist durch Hemmung der Talgproduktion wirksam bei besonders schweren Akne-Fällen, wirkt allerdings teratogen. Gleichwohl kann in bestimmten medizinisch indizierten Fällen die Verwendung der Retinoësäure bzw. ihrer Ester vorteilhaft sein und ist insofern in solchen Fällen als "unbedenklich" anzusehen.

Cyclodextrine sind aus 6, 7, 8 oder noch mehr α-1,4-verknüpften Glucoseeinheiten aufgebaut, wobei die Cyclohexaamylose (α-Cyclodextrin) sich durch die Struktur

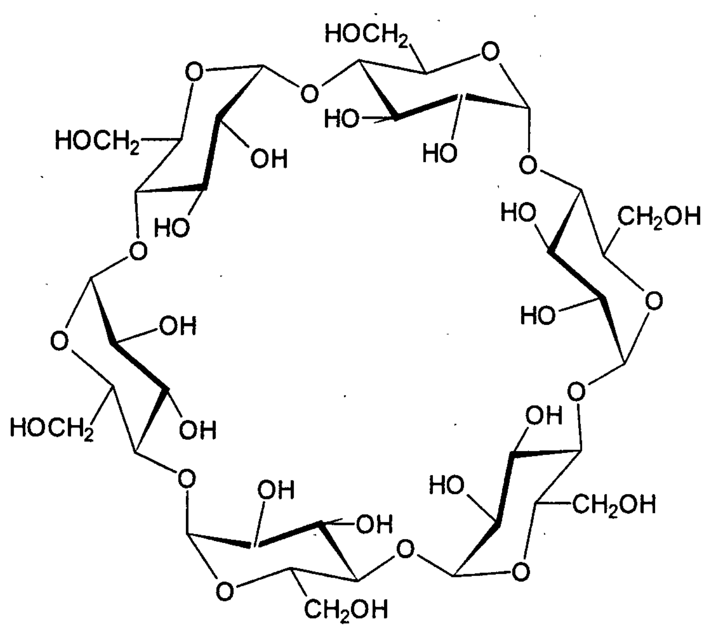

auszeichnet. Die Cycloheptaamylose (β-Cyclodextrin) zeichnet sich durch die Struktur

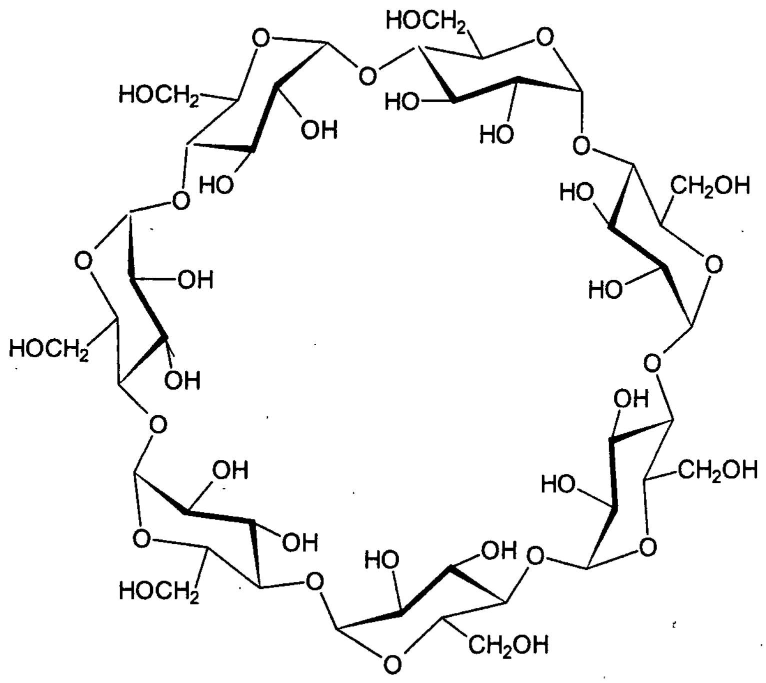

aus. Die Cyclooctaamylose (γ-Cyclodextrin) zeichnet sich durch die Struktur

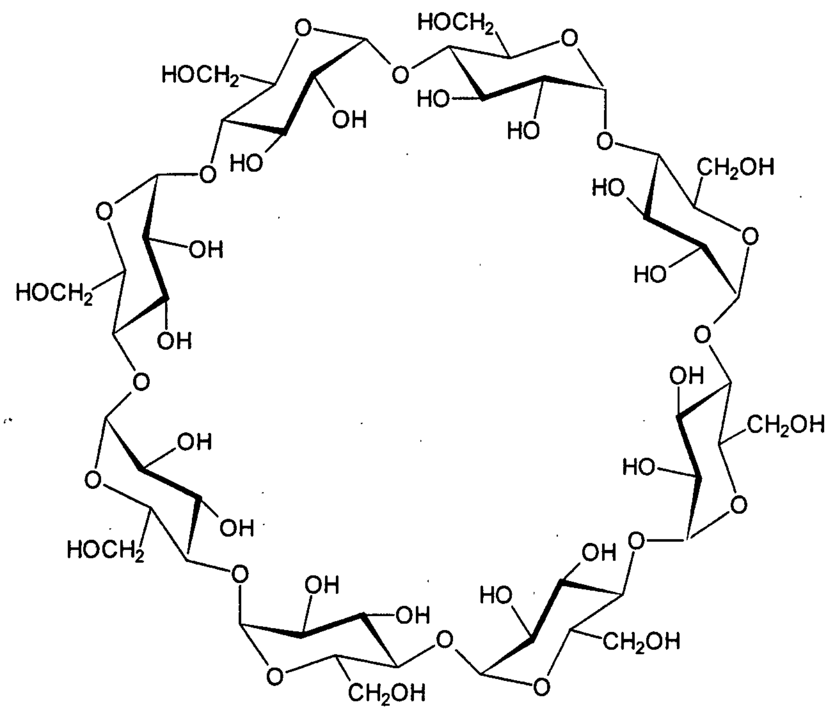

aus. Cyclodextrine können auch propoxyliert oder methyliert vorliegen. Insbesondere bedeutungsvoll sind Hydroxypropyl-β- Cyclodextrin, Hydroxypropyl-γ-Cyclodextrin und Methyl-β- Cyclodextrin.

Glycerylstearat ist ein bewährter Emulgator. Es kommt als nicht-selbstemulgierender oder selbstemulgierender Typ in den Handel. Dabei dient der nicht-selbstemulgierende Typ vorwiegend als W/O-Emulgator oder zur Stabilisierung von O/W-Emulsionen.

Retinoide sind oxidationempfindliche Wirkstoffe. Um sie stabil in Zubereitungen zu formulieren, wurden verschiedene Wege vorgeschlagen, beispielsweise molekulare Verkapselung. So lehrt die DE 19943678 Verwendung

von in Cyclodextrinen verkapselten Retinoiden in bestimmten Wirkstoffkombinationen.

Die Schrift WO 98/52536 beschreibt Zubereitungen zur Hautpflege, die Retinoide und bestimmte Konservierungsmittel enthalten.

Die Schriften WO 96/7396 beschreiben näher beschriebene Zubereitungen zur Hautpflege, die Retinoide und ein bestimmtes Stabilisatorsystem enthalten.

Die Schrift EP1023892 beschreibt die Stabilisierung von Retinoiden mit Hilfe von Zitronensäure und bestimmten Fettsäureglyceriden.

Die Schrift WO 98/25587 beschreibt ein näher bestimmtes Verfahren zur topischen Anwendung von Retinoiden mit lipophilen Antioxidantien.

Die Schrift WO 9814167 beschreibt kosmetische Zubereitungen, die Retinoide, öllösliche Antioxidantien und Emulgatoren und/oder Tenside enthält.

Die Schrift EP 676194 beschreibt kosmetische und/oder dermatologische Zubereitungen, die α-Hydroxysäuren, Salicylsäure oder deren Ester und ein Retinöid enthalten.

Die Schrift WO 9731620 beschreibt Zubereitungen zur Hautpflege auf der Basis von O/W-Emulsionen, die Retinoide und ein bestimmtes Emulgatorsystem enthalten.

Die Schrift EP 867175 beschreibt ein Verfahren zur Stabilisierung von Retinoiden mit Cyclodextrinen. Über den vorteilhaften Einsatz von derartig stabilisierten Retinoiden in kosmetischen und/oder dermatologischen W/O-Emulsionen wird nichts offenbart.

Die Schrift US 5543157 beschreibt bestimmte Arzneizubereitungen mit einem Gehalt an verschiedenen Wirkstoffen, darunter auch einzelne Retinoide, in Kombination mit Cyclodextrinen.

Die Schrift EP 756493 beschreibt Mehrkomponenten-Einschluß-Komplexe aus einem sauren Arzneimittel, einer Base und einem Cyclodextrin sowie die Verwendung derartiger Komplexe als Arzneimittel.

Weiterer Stand der Technik findet sich in WO 9855148, EP 579435.

Ausgehend vom hier dargestellten war Aufgabe der vorliegenden Erfindung, Retinoide so in kosmetische und/oder dermatologische Zubereitungen einzuarbeiten, daß der entsprechende Wirkstoff besser bioverfügbar ist und auch stabil in die Zubereitung eingearbeitet werden kann.

Es hat sich für den Fachmann nicht vorhersehbar herausgestellt, daß kosmetische und/oder dermatologische O/W-Emulsion mit einem Gehalt an Retinoiden, Glycerylstearat und Cyclodextrinen den Nachteilen des Standes der Technik abhelfen. Dabei ist es bevorzugt, wenn das oder die Cyclodextrine gewählt werden aus der Gruppe α-Cyclodextrin, β-Cyclodextrin, γ-Cyclodextrin, Hydroxypropyl-β-Cyciodextrin, Methyl-β-CD, Hydroxypropyl-γ-Cyclodextrin, sowie aus der Gruppe der Gemische aus Cyclodextrinen, welche mindestens zu 30 Gew.-%, bezogen auf das Gesamtgewicht des Cyclodextringemisches an γ-Cyclodextrin enthalten. Vorteilhaft beträgt der Gehalt an Cyclodextrinen 0,001 - 20,0 Gew.-%, bevorzugt 0,01 - 10,0 Gew.-%, besonders bevorzugt 0,1 - 5,0 Gew.-%, bezogen auf das Gesamtgewicht der Emulsion. Vorteilhaft ist es weiterhin, wenn das oder die Retinoide aus der Gruppe Retinol, Retinal, Retinylpalmitat, Retinylacetat und Retinsäure, besonders bevorzugt Retinol und Retinal gewählt werden. Es ist bevorzugt, den Gehalt an Retinoiden aus dem Bereich von 0,001 bis 2 Gew.-%, bevorzugt von 0,01 bis 1,0 Gew.-%, besonders bevorzugt von 0,05 bis 0,5 Gew.-% zu wählen, jeweils bezogen auf das Gesamtgewicht der Emulsion. Vorteilhaft ist außerdem ein Gehalt an Glycerylstearat von 0,01 bis 10 Gew.-%, bevorzugt von 0,1 bis 7,5 Gew.-%, besonders bevorzugt von 0,5 bis 5 Gew.-%, jeweils bezogen auf das Gesamtgewicht der Emulsion.

Von Vorteil ist weiterhin die Verwendung derartiger Emulsionen zur Behandlung und Prophylaxe der Symptome der intrinsischen und/oder extrinsischen Hautalterung, zur Behandlung und Prophylaxe der schädlichen Auswirkungen ultravioletter Strahlung auf die Haut, sowie zur Reduktion und/oder Vorbeugung der Bildung von Hautfältchen.

Die Erfindung umfaßt ebenso ein kosmetisches Verfahren zur Vorbeugung und/oder Bekämpfung von entzündlichen Hautzuständen und/oder Hautschutz bei empfindlich determinierter trockener Haut, zur Pflege der Haut nach Einwirkung von Sonnenlicht und/oder zur Verminderung der Nachreaktion der Haut auf die Einwirkung von UV-Strahlung, sowie zur Immunstimulation der Haut, bevorzugt zur Immunstimulation im Sinne einer Behandlung der verletzten Haut, besonders bevorzugt zur Behandlung von Wunden. Bei diesen Verfahren werden die beschriebenen Emulsionen mit dem betroffenen Bereich der Haut in Kontakt gebracht werden.

Die Emulsionen gemäß der Erfindung sind in jeglicher Hinsicht überaus befriedigende Präparate. Es war für den Fachmann nicht vorauszusehen, daß die Zubereitungen gemäß der Erfindung

- in wirksamen Konzentrationen Retinoide bioverfügbar bereitstellen,
- dabei die chemische Stabilität der Retinoide in den Zubereitungen verbessern,
- eine wesentlich bessere Verträglichkeit der Retinoide gewährleisten,
- besser die Barriereeigenschaften der Haut erhalten oder wiederherstellen,
- besser der Hautaustrocknung entgegenwirken,
- die Haut besser vor Umwelteinflüssen schützen

als die Zubereitungen des Standes der Technik.

Bei Anwendung der erfindungsgemäß verwendeten Emulsionen ist in überraschender Weise eine wirksame Behandlung, aber auch eine Prophylaxe

- von defizitären, sensitiven oder hypoaktiven Hautzuständen oder defizitären, sensitiven oder hypoaktiven Zustände von Hautanhangsgebilden,
- von umweltbedingten (Rauchen, Smog, reaktive Sauerstoffspecies, freie Radikale) und insbesondere lichtbedingten negativen Veränderungen der Haut und der Hautanhangsgebilde,
- von lichtbedingten Hautschäden,
- von Symptomen der intrinsischen und/oder extrinsischen Hautalterung sowie der schädlichen Auswirkungen ultravioletter Strahlung auf die Haut,
- von trockenen Hautzuständen und Hornschichtbarrierestörungen,
- von entzündlichen Hautzuständen sowie atopischem Ekzem, seborrhoischem Ekzem, polymorpher Lichtdermatose, Psoriasis, Vitiligo
- möglich.

Die erfindungsgemäße Emulsion dient aber auch in überraschender Weise

- zur Beruhigung von empfindlicher oder gereizter Haut,
- zur Stimulation der intrazellulären DNA-Synthese, insbesondere bei defizitären oder hypoaktiven Hautzuständen,
- zum Hautschutz bei empfindlich determinierter trockener Haut,
- zur Stärkung des desoxidativen Hauteigenschutzes,
- Reduktion und/oder Vorbeugung von Hautfältchen,
- zur Steigerung der hauteigenen Schutz- und Reparaturmechanismen (beispielsweise für dysfunktionelle Enzyme, DNA, Lipide, Proteine),
- zur Vor- und Nachbehandlung bei topischer Anwendung von Laser- und Abschleifbehandlungen, die z. B. der Reduzierung von Hautfalten und Narben dienen, um den resultierenden Hautreizungen entgegenzuwirken und die Regenerationsprozesse in der verletzten Haut zu fördern.

Die in den Emulsionen gemäß der Erfindung enthaltenen Wirkstoffkombinationen wirken in all diesen Verwendungen synergistisch in bezug auf die einzelnen Komponenten.

Erfindungsgemäß vorteilhaft ist der Zusatz von mindestens eines weiteren, oben nicht genannten Cyclodextrins und/oder Cyclodextrinderivates, gewählt aus der Gruppe der nativen Cyclodextrine α-, β- und γ-Cyclodextrin, ganz oder teilweise an den Hydroxylgruppen veretherten und/oder veresterten und/oder anders derivatisierten α-, β- und γ-Cyclodextrine, insbesondere das Hydroxypropyl-β-Cyclodextrin, Hydroxypropyl-γ-Cyclodextrin sowie das Methyl-β-Cyclodextrin.

Erfindungsgemäß ist dabei eine Konzentration von 0,001 bis 20% und insbesondere von 0,1 bis 5 Gewichts-% bezogen auf das Gesamtgewicht der Zubereitung an mindestens einer Cyclodextrinspezies und/oder dessen Derivaten vorteilhaft.

Erfindungsgemäß besonders bevorzugt ist γ-Cyclodextrin.

Erfindungsgemäß vorteilhaft ist die Verwendung von Cyclodextrinen und/oder Cyclodextrinderivaten zur Erhöhung der Löslichkeit von Retinoiden.

Weiterhin vorteilhaft ist die Verwendung von Cyclodextrinen und/oder Cyclodextrinderivaten zur Verbesserung der biologischen Wirksamkeit von Retinoiden.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren,

Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders bevorzugt sind α-Liponsäuren, Ubichinon, Q10 und α-Glycosylrutin;

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut voroxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittelund Arzneimittelgesetz).

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z.B.:

- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisöpentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- 2,4,6-Trianilino-(p-carbo-2'-ethyl-1'-hexyloxy)-1,3,5-triazin.

Vorteilhafte wasserlösliche UVB-Filter sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entsprechenden 10-Sulfato-verbindungen, beispielsweise das entsprechende Natrium-, Kalium- oder Triethanolam- monium-Salz), auch als Benzol-1,4-di(2-oxo-3-bornylidenmethyl-10-Sulfonsäure bezeichnet.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, UVA-Filter einzusetzen, die üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Weiterhin vorteilhafte UVA-Filter entstammen der Gruppe der Triazine, so z.B. das 2,4-Bis-{[4-(2-Ethyl-hexyloxy)-2-hydroxy]-phenyl}-6-(4-methoxyphenyl)-1,3,5-triazin (Handelsbezeichnung Tinosorb® S) sowie der Gruppe der Triazole, wie z.B. das 2,2'-Methylen-bis- [6-2H-benzotriazol-2yl]-4-(1,1,3,3-tetramethylbutyl)phenol) (Handelsbezeichnung Tinosorb® M). Ein vorteilhafter wasserlöslicher UVA-Filter stellt das 2'-bis-(1,4-Phenylen)-1H-benzimidazol-4,6-disulfonsäure-Natriumsalz dar (Handelsbezeichnung Neo Heliopan AP®).

Es können die für die UVB-Kombination verwendeten Mengen eingesetzt werden.

Erfindungsgemäße kosmetische und dermatologische Zubereitungen enthalten vorteilhaft außerdem anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliziums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenngleich nicht zwingend, wenn die anorganischen Pigmente in hydrophober Form vorliegen, d.h., dass sie oberflächlich wasserabweisend behandelt sind. Diese Oberflächenbehandlung kann darin bestehen, dass die Pigmente nach an sich bekannten Verfahren mit einer dünnen hydrophoben Schicht versehen werden.

Eines solcher Verfahren besteht beispielsweise darin, dass die hydrophobe Oberflächenschicht nach einer Rektion gemäß

$$n\ TiO_2 + m\ (RO)_3\ Si\text{-}R' \text{ -> } n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma TAYCA, ferner M 160 von der Firma Kemira sowie T 805 von der Firma Degussa erhältlich.

Die Prophylaxe bzw. die kosmetische oder dermatologische Behandlung mit dem erfindungsgemäß verwendeten Wirkstoff bzw. mit den kosmetischen oder topischen dermatologischen Zubereitungen mit einem wirksamen Gehalt an erfindungsgemäß verwendetem Wirkstoff erfolgt in der üblichen Weise, und zwar dergestalt, dass der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen aufgetragen wird.

Vorteilhaft kann der erfindungsgemäß verwendete Wirkstoff eingearbeitet werden in übliche kosmetische und dermatologische Zubereitungen, welche in verschiedenen Formen vorliegen können. So können sie z.B. eine Lösung, eine Emulsion vom TypWasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Erfindungsgemäße Emulsionen im Sinne der vorliegenden Erfindung, z.B. in Form einer Crème, einer Lotion, einer kosmetischen Milch, eines Emulsions-Schaumes aus einem Aerosolbehälter sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wässrige Systeme bzw. Tensidzubereitungen zur Reinigung der Haut und der Haare einzufügen.

Die Lipidphase der erfindungsgemäßen kosmetischen oder dermatologischen Emulsionen kann vorteilhaft gewählt werden aus folgender Substanzgruppe:

- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;

- Alkylbenzoate;
- Silikonöle wie Dimethylpolysiloxane, Diethylpolysiloxane, Diphenylpolysiloxane sowie Mischformen daraus.

Die Öle werden vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der GruppeIsopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyidodecylpaimitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Öctyldodecanol, Isotridecylisononanoat, Isoeicosan, 2-Ethylhexylcocoat, $C_{12-15}$-Alkylbenzoat, Capryl-Caprinsäure-triglycerid, Dicaprylylether, Dicaprylylcarbonat.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an zyklischen oder linearen Silikonölen aufweisen oder vollständig aus solchen Ölen bestehen, wobei allerdings bevorzugt wird, außer dem Silikonöl öder den Silikonölen einen zusätzlichen Gehalt an anderen Ölphasenkomponenten zu verwenden. Solche Silicone oder Siliconöle können als Monomere vorliegen, welche in der Regel durch Strukturelemente charakterisiert sind, wie folgt:

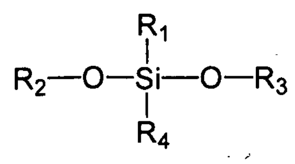

Als erfindungsgemäß vorteilhaft einzusetzenden linearen Silicone mit mehreren Siloxyleinheiten werden im allgemeinen durch Strukturelemente charakterisiert wie folgt:

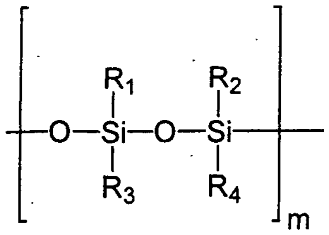

wobei die Siliziumatome mit gleichen oder unterschiedlichen Alkylresten und/oder Arylresten substituiert werden können, welche hier verallgemeinernd durch die Reste $R_1$ - $R_4$ dargestellt sind (will sagen, dass die Anzahl der unterschiedlichen Reste nicht notwendig auf bis zu 4 beschränkt ist). m kann dabei Werte von 2 - 200.000 annehmen.

Erfindungsgemäß vorteilhaft einzusetzende zyklische Silicone werden im allgemeinen durch Strukturelemente charakterisiert, wie folgt

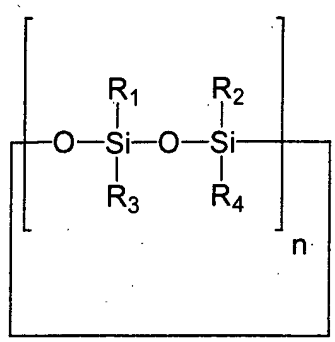

wobei die Siliziumatome mit gleichen oder unterschiedlichen Alkylresten und/oder Arylresten substituiert werden können, welche hier verallgemeinernd durch die Reste $R_1$ - $R_4$ dargestellt sind (will sagen, dass die Anzahl der unterschiedlichen Reste nicht notwendig auf bis zu 4 beschränkt ist). n kann dabei Werte von 3/2 bis 20 annehmen. Gebrochene Werte für n berücksichtigen, dass ungeradzahlige Anzahlen von Siloxylgruppen im Zyklus vorhanden sein können.

Vorteilhaft wird Cyclomethicon (z.B. Decamethylcyclopentasiloxan) als erfindungsgemäß zu verwendendes Silikonöl eingesetzt. Aber auch andere Silikonöle sind vorteilhaft im Sinne der vorliegenden Erfindung zu verwenden, beispielsweise Undecamethylcyclotrisiloxan, Polydimethylsiloxan, Poly(methylphenylsiloxan), Cetyldimethicon, Behenoxydimethicon.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxan-polyalkyl-polyether-copolymere wie das Cetyl-Dimethicon-Copolyol, das (Cetyl-Dimethicon-Copolyol (und) Polyglyceryl-4-Isostearat (und) Hexyllaurat)

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyloder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliziumdioxid, Aluminiumsilikate.

Erfindungsgemäße als Emulsionen vorliegenden Zubereitungen enthalten insbesondere vorteilhaft ein oder mehrere Hydrokolloide. Diese Hydrokolloide können vorteilhaft gewählt werden aus der Gruppe der Gummen, Polysaccharide, Cellulosederivate, Schichtsilikate, Polyacrylate und/oder anderen Polymeren.

Erfindungsgemäße als Hydrogele vorliegenden Zubereitungen enthalten ein oder mehrere Hydrokolloide. Diese Hydrokolloide können vorteilhaft aus der vorgenannten Gruppe gewählt werden.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können vorteilhaft auch Kieselsäuregele verwendet werden.
Unter den Polyacrylaten befinden sich z.B. Carbopol Typen der Firma Goodrich (Carbopol 980, 981, 1382, 5984, 2984, ETD 2001, ETD 2020, ETD 2050, Ultrez 10 oder Pemulen TR1 & TR2).
Unter den Polymeren befinden sich z.B. Polyacrylamide (Seppigel 305), Polyvinylalkohole, PVP, PVP / VA Copolymere, Polyglycole.
Erfindungsgemäße als Emulsionen vorliegenden Zubereitungen enthalten einen oder mehrere Emulgatoren. Diese Emulgatoren können vorteilhaft gewählt werden aus der Gruppe der nichtionischen, anionischen, kationischen oder amphoteren Emulgatoren.
Unter den nichtionischen Emulgatoren befinden sich

a) Partialfettsäureester und Fettsäureester mehrwertiger Alkohole und deren ethoxylierte Derivate (z. B. Glycerylmonostearate, Sorbitanstearate, Glycerylstearylcitrate, Sucrosestearate)

b) ethoxylierte Fettalkohole und Fettsäuren

c) ethoxilierte Fettamine, Fettsäureamide, Fettsäurealkanolamide

d) Alkylphenolpolyglycolether (z.B. Triton X)

e) Zuckerderivate (Ester und/oder Ether von Glucose, Saccharose und anderen Zuckern; z.B. Alkylpolyglycoside wie Polyglyceryl-3-Methylglucose-Distearat, Methylglucosesesquistearat)

Unter den anionischen Emulgatoren befinden sich

a) Seifen (z.B. Natriumstearat)

b) Fettalkoholsulfate

c) Mono-, Di- und Trialkylphosphosäureester und deren Ethoxylate

Unter den kationischen Emulgatoren befinden sich

a) quaternäre Ammoniumverbindungen mit einem langkettigen aliphatischen Rest z.B. Distearyldimonium Chloride

Unter den amphoteren Emulgatoren befinden sich

a) Alkylamininoalkancarbonsäuren

b) Betaine, Sulfobetaine

c) Imidazolinderivate

Weiterhin gibt es natürlich vorkommende Emulgatoren, zu denen Bienenwachs, Wollwachs, Lecithin und Sterole gehören.
O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:

- der Fettalkoholethoxylate
- der ethoxylierten Wollwachsalkohole,
- der Polyethylenglycolether der allgemeinen Formel R-O-(-CH2-CH2-O-)n-R',
- der Fettsäureethoxylate der allgemeinen Formel R-COO-(-CH2-CH2-O-)n -H,
- der veretherten Fettsäureethoxylate der allgemeinen Formel R-COO-(-CH2-CH2-O-)n -R',
- der veresterten Fettsäureethoxylate der allgemeinen Formel R-COO-(-CH2-CH2-O-)n -C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel R-O-(-CH2-CH2-O-)n-CH2-COOHnd n eine Zahl von 5 bis

30 darstellen,

- der Polyoxyethylensorbitolfettsäureester,
- der Alkylethersulfate der allgemeinen Formel R-O-(-CH2-CH2-O-)n-SO3-H
- der Fettalkoholpropoxylate der allgemeinen Formel R-O-(-CH2-CH(CH3)-O-)n-H,
- der Polypropylenglycolether der allgemeinen Formel R-O-(-CH2-CH(CH3)-O-)n-R',
- der propoxylierten Wollwachsalkohole,
- der veretherten Fettsäurepropoxylate R-COO-(-CH2-CH(CH3)-O-)n-R',
- der veresterten Fettsäurepropoxylate der allgemeinen Formel R-COO-(-CH2-CH(CH3)-O-)n-C(O)-R',
- der Fettsäurepropoxylate der allgemeinen Formel R-COO-(-CH2-CH(CH3)-O-)n-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel R-O-(-CH2-CH(CH3)O-)n-CH2-COOH
- der Alkylethersulfate bzw. die diesen Sulfaten zugrundeliegenden Säuren der allgemeinen Formel R-O-(-CH2-CH(CH3)-O-)n-SO3-H
- der Fettalkoholethoxylate/pröpoxylate der allgemeinen Formel R-O-Xn-Ym-H,
- der Polypropylenglycolether der allgemeinen Formel R-O-Xn-Ym-R',
- der veretherten Fettsäurepropoxylate der allgemeinen Formel R-COO-Xn-Ym-R',
- der Fettsäureethoxylate/propoxylate der allgemeinen Formel R-COO-Xn-Ym-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol-(18)stearylether (Steareth-18), Polyethylenglycol(19)stearylether (Steareth-19), Polyethylenglycol(20)stearylether (Steareth-20),

Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19-), Polyethylenglycol(20)isostearylether (Isosteareth-20),

Polyethylenglycol(13)cetylether (Ceteth-13), Polyethylenglycol(14)cetylether (Ceteth-14), Polyethylenglycol(15) cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20) cetylether (Ceteth-20),

Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17) isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),

Polyethylenglycol(12)oleylether (Oleth-12), Polyethylenglycol(13)oleylether (Oleth-13), Polyethylenglycol(14) oleylether (Oleth-14), Polyethylenglycol(15)oleylether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)-cetylstearylether (Ceteareth-19), Polyethylenglycol(20)cetylstearylether (Ceteareth-20),

Es ist ferner von Vorteil, die Fettsäureethoxylate aus folgender Gruppe zu wählen:

Polyethylenglycol(20)stearat, Polyethylenglycol(21)stearat, Polyethylenglycol(22)stearat, Polyethylenglycol(23) stearat, Polyethylenglycol(24)stearat, Polyethylenglycol(25)stearat,

Polyethylenglycol(12)isostearat, Polyethylenglycol(13)isostearat, Polyethylenglycol(14)-isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol-(22)isostearat, Polyethylenglycol(23)isostearat, Polyethylenglycol(24)isostearat, Polyethylenglycol (25)isostearat,

Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20) glyceryllaurat, Polyethylenglycol(21)glyceryllaurat, Polyethylenglycol(22)glyceryllaurat, Polyethylenglycol(23)glyceryllaurat, Polyethylenglycol(6)-glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/ oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Als Hautbefeuchtungsmittel lassen sich vorteilhaft Glycerin, Panthenol, Chitosan, Fucogel, Propylenglycol, Dipropylenglycol, Butylenglycol, Mannitol, Milchsäure, Natriumpyrolidoncarbonsäure, Hyaluronsäure, Salze der angegebenen Säuren sowie Glycin, Harnstoff und Salze von Metallen der ersten und zweiten Hauptgruppe verwenden.

Besonders geeignet sind Glycerin, Milchsäure, Butylenglycol, Harnstoff, Hyaluronsäure.

Der Gehalt an Hautbefeuchtungsmitteln beträgt vorteilhaft 1 Gew.-% bis 30 Gew.-%, vorzugsweise 2 bis 25 Gew.-%, insbesondere 5 bis 20 Gew.-%, bezogen auf das Gesamtgewicht der Zubereitungen.

Erfindungsgemäße Zubereitungen können, zumal wenn kristalline oder mikrokristalline Festkörper, beispielsweise anorganische Mikropigmente in die erfindungsgemäßen Zubereitungen eingearbeitet werden sollen, auch anionische, nichtionische und/oder amphotere Tenside enthalten. Tenside sind amphiphile Stoffe, die organische, unpolare Substanzen in Wasser lösen können.

Bei den hydrophilen Anteilen eines Tensidmoleküls handelt es sich meist um polare funktionelle Gruppen,

beispielweise $-COO^-$, $-OSO_3^{2-}$, $-SO_3^-$, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:

- anionische Tenside,
- kationische Tenside,
- amphotere Tenside und
- nichtionische Tenside.

Anionische Tenside weisen als funktionelle Gruppen in der Regel Carboxylat-, Sulfatoder Sulfonatgruppen auf. In wässriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

$RNH_2^+CH_2CH_2COOH\ X^-$ (bei pH=2) $X^-$ = beliebiges Anion, z.B. $Cl^-$

$RNH_2^+CH_2CH_2COO^-$ (bei pH=7)

$RNHCH_2CH_2COO^-\ B^+$ (bei pH=12) $B^+$ = beliebiges Kation, z.B. $Na^+$

Typisch für nicht-ionische Tenside sind Polyether-Ketten. Nicht-ionischeTenside bilden in wässrigem Medium keine Ionen.

Vorteilhaft ist ferner die Verwendung einer Kombination von anionischen und/oder amphoteren Tensiden mit einem oder mehreren nicht-ionischen Tensiden.

Die oberflächenaktive Substanz kann in einer Konzentration zwischen 1 und 30 Gew.-% in den erfindungsgemäßen Zubereitungen vorliegen, bezogen auf das Gesamtgewicht der Zubereitungen.

Es ist dem Fachmanne natürlich bekannt, dass anspruchsvolle kosmetische Zusammensetzungen zumeist nicht ohne die üblichen Hilfs- und Zusatzstoffe denkbar sind. Die erfindungsgemäßen kosmetischen Zubereitungen können daher kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, weitere Vitamine, Mittel zum Verhindern des Schäumens, Farbstoffe, Pigmente mit färbender Wirkung, Verdickungsmittel, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

<u>Beispielrezepturen:</u>

Beispiele 1-4: O/W-Cremes, Beispiel 5: W/O/W-Creme

| Beispiel Nummer | 1 | 2 | 3 | 4 | 5 |
|---|---|---|---|---|---|
| Glycerylsterat, selbstemulgierend | 3 | 5 | 2 | 6 | 3 |

(fortgesetzt)

| Beispiel Nummer | 1 | 2 | 3 | 4 | 5 |
|---|---|---|---|---|---|
| PEG-40-Stearat | | | 1 | | 1 |
| Myristylmyristat | | | | | |
| Behenylalkohol | 1 | | | | |
| Stearylalcohol | | | | | |
| Cetearylalkohol | 1 | | | 2 | |
| Cetylalcohol | 1 | 2 | 3 | | 2 |
| Hydrierte Cocosfettglyceride (Hydrogenated Coco Glycerides) | 1 | | | | |
| Sheabutter | 1 | | 3 | | 1 |
| C12-15 Alkylbenzoat | | 2 | 2 | | 3 |
| Butylenglycol Dicaprylat/Dicaprat | 1 | | | 1 | |
| Capryisäure/Caprinsäure Triglycerid | 2 | 5 | 1 | 2 | 3 |
| Hydriertes Polydecen | | 1 | 1 | | |
| Ethylhexylkokosfettsäureester | | | | | 1 |
| Octyldodecanol | | | 1 | | |
| Mineralöl | | 1 | | | |
| Vaseline | | | 1 | | |
| Octamethyltetrasiloxan (Cyclomethicon) | 2 | 2 | 4 | 3 | 4 |
| Dimethylpolysiloxan (Dimethicon) | | | | 1 | |
| Dicaprylylether | 1 | | | | |
| Dicarprylylcarbonat | | | | 3 | |
| Sqalan | | 1 | 1 | | |
| Ricinusöl | | | | | 5 |
| Polydecen | | | 1 | | |
| $TiO_2$ | 1 | | 0.5 | | |
| Ethylhexylmethoxycinnamat | 2 | 2 | | | |
| Ethylhexyltriazon | | | | 2 | |
| Ethylhexylcyanodiphenylacrylat (Octocrylen) | | | 2 | | 5 |
| Butylmethoxydibenzoylmethan | | 1 | | | |
| Bis-Ethylhexyloxyphenolmethoxyphenyltriazine | | | | 1 | 1 |
| Retinol | 0,05 | | 0,05 | 0,1 | 0,10 |
| Retinylpalmitat | 0,1 | | | 0,2 | |
| Retinol-γ-Cyclodextrin-Komplex | | 1,0 | | | |
| Biotin | | | | | 0,05 |
| Ascorbinsäure | | | | 0,10 | |
| Tocopherylacetat | | 0,5 | | | 1 |
| β-Cyclodextrin | | | | | |
| γ-Cyclodextrin | 2,0 | | 0,5 | 1,0 | 0.8 |
| Trinatrium EDTA | | 0.1 | | | 0,2 |

(fortgesetzt)

| Beispiel Nummer | 1 | 2 | 3 | 4 | 5 |
|---|---|---|---|---|---|
| Iminodisuccinat, Natriumsalz | 0,2 | | 0,1 | 0,3 | |
| Phenoxyethanol | 0,3 | | 0,3 | 0,2 | 0,2 |
| p-Hydroxybenzoesäurealkylester (Paraben) | 0,5 | 0,3 | 0,2 | 0,3 | 0,3 |
| Hexamidindiisethionat | | 0,04 | | | 0,1 |
| Diazolidinylharnstoff | | | 0,1 | 0,2 | 0,1 |
| 1,3-Dimethylol-5,5-dimethylhydantoin (DMDM Hydantoin) | | | | 0,1 | |
| Iodopropynylbutylcarbamat | | 0,3 | | | |
| Hexandiol | 2 | | | 1 | |
| Ethanol denaturiert | | | | 1 | |
| Xanthan Gummi | 0,1 | | | 0,1 | |
| Polyacrylsäure (Carbomer) | 0,1 | 0,1 | 0,1 | | 0,1 |
| Polyacrylamid | | | | | |
| Glycerin | 4 | 10 | 8 | 7 | 8 |
| Propylenglycol | 2 | | | | |
| wasser- und/oder öllösliche Farbstoffe | 0,1 | | | | |
| Additive (Distärke-phosphat, BHT, Talkum, Aluminium stärke octenylsuccinat, Aluminiumstearat, Natrium Chlorid) | 1 | 0,5 | 0,1 | 0,2 | 1,5 |
| Pflanzenextrakte, pflanzliche Öle | 2 | | | | |
| Parfüm | q.s. | q.s. | q.s. | q.s. | q.s. |
| Wasser | Ad 100 | Ad 100 | Ad 100 | Ad 100 | Ad 100 |

**Patentansprüche**

1. Kosmetische und/oder dermatologische O/W-Emulsion mit einem Gehalt an Retinoiden, Glycerylstearat und Cyclodextrinen.

2. Emulsion nach Anspruch 1 **dadurch gekennzeichnet, daß** das oder die Cyclodextrine gewählt werden aus der Gruppe α-Cyclodextrin, β-Cyclodextrin, γ-Cyclodextrin, Hydroxypropyl-β-Cyclodextrin, Methyl-β-CD, Hydroxypropyi-γ-Cyciodextrin, sowie aus der Gruppe der Gemische aus Cyclodextrinen, welche mindestens zu 30 Gew.-%, bezogen auf das Gesamtgewicht des Cyclodextringemisches an γ-Cyclodextrin enthalten.

3. Emulsion nach mindestens einem der vorangehenden Ansprüche mit einen Gehalt an Cyclodextrinen von 0,001 - 20,0 Gew.-%, bevorzugt 0,01 - 10,0 Gew.-%, besonders bevorzugt 0,1 - 5,0 Gew.-%, bezogen auf das Gesamtgewicht der Emulsion.

4. Emulsion nach mindestens einem der vorangehenden Ansprüche **dadurch gekennzeichnet, daß** das oder die Retinoide aus der Gruppe Retinol, Retinal, Retinylpalmitat, Retinylacetat und Retinsäure, besonders bevorzugt Retinol und Retinal gewählt werden.

5. Emulsion nach mindestens einem der vorangehenden Ansprüche **dadurch gekennzeichnet, daß** der Gehalt an Retinoiden aus dem Bereich von 0,001 bis 2 Gew.-%, bevorzugt von 0,01 bis 1,0 Gew.-%, besonders bevorzugt von 0,05 bis 0,5 Gew.-% gewählt wird, jeweils bezogen auf das Gesamtgewicht der Emulsion.

6. Emulsion nach mindestens einem der vorangehenden Ansprüche **dadurch gekennzeichnet, daß** der Gehalt an Glycerylstearat aus dem Bereich von 0,01 bis 10 Gew.-%, bevorzugt von 0,1 bis 7,5 Gew.-%, besonders bevorzugt

von 0,5 bis 5 Gew.-% gewählt wird, jeweils bezogen auf das Gesamtgewicht der Emulsion.

**7.** Verwendung von Emulsionen nach mindestens einem der vorangehenden Ansprüche zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut.

**8.** Verwendung von Emulsionen nach mindestens einem der Ansprüche 1 bis 6 zur Reduktion und/oder Vorbeugung der Bildung von Hautfältchen.

**9.** Kosmetisches Verfahren zur Vorbeugung und/oder Bekämpfung von entzündlichen Hautzuständen und/oder Hautschutz bei empfindlich determinierter trockener Haut, **dadurch gekennzeichnet, daß** das eine Emulsion nach mindestens einem der Ansprüche 1 bis 6 mit dem betroffenen Bereich in Kontakt gebracht wird.

**10.** Kosmetisches Verfahren zur Pflege der Haut nach Einwirkung von Sonnenlicht und/oder zur Verminderung der Nachreaktion der Haut auf die Einwirkung von UV-Strahlung, **dadurch gekennzeichnet, daß** das eine Emulsion nach mindestens einem der Ansprüche 1 bis 6 mit dem betroffenen Bereich in Kontakt gebracht werden.

**11.** Kosmetisches Verfahren zur Immunstimulation der Haut, bevorzugt zur Immunstimulation im Sinne einer Behandlung der verletzten Haut, besonders bevorzugt zur Behandlung von Wunden, **dadurch gekennzeichnet, daß** das eine Emulsion nach mindestens einem der Ansprüche 1 bis 6 mit dem betroffenen Bereich in Kontakt gebracht werden.